# EUROPEAN PATENT SPECIFICATION

(11) **EP 3 638 212 B1**
(45) Date of publication and mention of the grant of the patent: **01.05.2024**
(21) Application number: 18816473.5
(22) Date of filing: 14.06.2018
(51) Int. Cl.: A61K 9/50, A61K 31/519, B01J 13/00, C07D 471/04

(54) **METHODS FOR PRODUCING PARTICLES OF AN ACTIVE INGREDIENT**
VERFAHREN ZUR HERSTELLUNG VON PARTIKELN EINES WIRKSTOFFS
PROCÉDÉS DE PRODUCTION DE PARTICULES D'UN INGRÉDIENT ACTIF

(30) Priority: 15.06.2017 US 201762519983 P
(43) Date of publication of application: 22.04.2020
(73) Proprietor: Savior Lifetec Corporation, Miaoli County 350 (TW)
(72) Inventor: PAN, Shih-Hsie, Princeton Junction NJ 08550 (US); CHEN, Yan-Ming, Kaohsiung City 820 (TW); KU, Mannching, Sherry, Thiells NY 10984 (US); LIN, Chia-Hsin, Miaoli County 350 (TW)
(74) Representative: Lang, Christian
(86) International application number: PCT/US2018/037431
(87) International publication number: WO 2018/232053

(56) References cited:
- WO-A1-2015/152145
- WO-A1-2016/116831
- WO-A2-2005/009375
- WO-A2-2013/046225
- WO-A2-2016/199170
- US-A1- 2004 242 619

## Description

### BACKGROUND OF THE INVENTION

### 1. FIELD OF THE INVENTION

The present disclosure relates to pharmaceutical field, and more particularly, to a method of producing particles of an active ingredient, especially paliperidone palmitate or ascorbic acid.

### 2. DESCRIPTION OF RELATED ART

One way of achieving fast release of a therapeutic agent *in vivo* is through the use of particles, such as nanoparticles and/or microparticles. These particles can be administered through different routes, such as oral and parenteral routes. Particles thus can be inhaled or injected (e.g., intravenously, subcutaneously or intramuscularly). Due to the easy-to-use property of particles, intensive research has been drawn to the development of improved methods of producing particles of a therapeutic agent.

Document WO 2005/009375 A2 discloses a method for preparing small spherical particles of an active ingredient, wherein the method comprises (a) preparing a solution of the active ingredient in a first solvent; (b) adding a second solvent; (c) spreading the obtained solution onto a surface to form a thin film; and (d) evaporating the solvents from the thin film to form small spherical particles of the active ingredient. Document WO 2016/199170 A2 teaches a method for producing paliperidone palmitate particles, which uses a warm, first solvent and a second solvent, wherein the examples therein relate to a process, in which both of the first and second solvent are ethanol. Document WO 2016/116831 A1 teaches another process for producing paliperidone palmitate particles, in which a first solvent to be used is an alcohol selected from a group comprising of methanol, ethanol, n-propanol, isopropanol, n-butanol, isobutanol, tert-butanol or mixture thereof, and wherein a second solvent to be used is water. Document WO 2015/152145 A1 discloses the preparation of 2-O-α-D-glucosyl-L-ascorbic acid crystals.

Inventors of the present disclosure unexpectedly identify an improved method of producing particles, particularly, nanoparticles and microparticles, of an active agent. The present method takes advantages in the differences between solubilities of the therapeutic agent in two miscible solvent systems, so that the therapeutic agent soluble in one solvent system precipitates when another solvent is introduced therein. The particles thus produced are not only small in size, but also exhibit relatively uniform size distribution, these particles are thus suitable for use in medical formulation.

### SUMMARY

The following description presents a simplified summary of the disclosure in order to provide a basic understanding to the reader. This summary is not an extensive overview of the disclosure and it does not identify key/critical elements of the present invention or delineate the scope of the present invention. Its sole purpose is to present some concepts disclosed herein in a simplified form as a prelude to the more detailed description that is presented later.

In one aspect, the present disclosure is directed to a method of producing particles of an active ingredient according to claim 1. The present method comprises the steps of:
(a) forming a first solution by dissolving the active ingredient in a first solvent;
(b) forming the particles of the active ingredient by mixing a second solvent with the first solution to produce an emulsified solution;
wherein, the second solvent is miscible with the first solvent, but is immiscible with the first solution,
wherein either the active ingredient is paliperidone palmitate, the first solvent is toluene and the second solvent is hexane or heptane; or
the active ingredient is ascorbic acid, the first solvent is methanol and the second solvent is water; or the first solvent is tetrahydrofuran and the second solvent is heptane.

In some embodiments, the method of present invention further comprises step (c): removing the first and second solvents from the emulsified solution to produce a powder of the particles of the active ingredient.

According to other embodiments, the method further comprises step (a-1): filtering the first solution before the step (b). In one specific embodiment, the first solution is filtered through a filter having a pore size of 0.22 µm.

Many of the attendant features and advantages of the present disclosure will become better understood with reference to the following detailed description considered in connection with the accompanying drawings.

In accordance with common practice, the various described features/elements are not drawn to scale but instead are drawn to best illustrate specific features/elements relevant to the present invention. Also, like reference numerals and designations in the various drawings are used to indicate like elements/parts.

### BRIEF DESCRIPTION OF THE DRAWINGS

The present description will be better understood from the following detailed description read in light of the accompanying drawings, where:

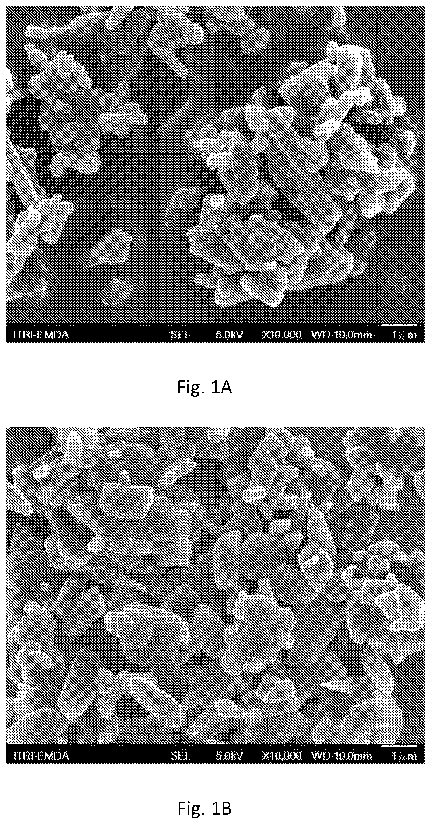

Figure 1A is a Scanning Electron Microscope (SEM) image illustrating the crystalline of nanoparticles without milling; and
Figure 1B is a Scanning Electron Microscope (SEM) image illustrating the crystalline of nanoparticles with milling.

### DESCRIPTION

The detailed description provided below is not intended to represent the only forms in which the present example may be constructed or utilized. The description sets forth the functions of the example and the sequence of steps for constructing and operating the example. However, the same or equivalent functions and sequences may be accomplished by different examples.

### 1. Definitions

For convenience, certain terms employed in the specification, examples and appended claims are collected here. Unless otherwise defined herein, scientific and technical terminologies employed in the present disclosure shall have the meanings that are commonly understood and used by one of ordinary skill in the art. Also, unless otherwise required by context, it will be understood that singular terms shall include plural forms of the same and plural terms shall include the singular. Specifically, as used herein and in the claims, the singular forms "a" and "an" include the plural reference unless the context clearly indicates otherwise. Also, as used herein and in the claims, the terms "at least one" and "one or more" have the same meaning and include one, two, three, or more.

Unless otherwise required by context, it will be understood that singular terms shall include plural forms of the same and plural terms shall include the singular. Also, as used herein and in the claims, the terms "at least one" and "one or more" have the same meaning and include one, two, three, or more. Furthermore, the phrases "at least one of A, B, and C", "at least one of A, B, or C" and "at least one of A, B and/or C," as use throughout this specification and the appended claims, are intended to cover A alone, B alone, C alone, A and B together, B and C together, A and C together, as well as A, B, and C together.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in the respective testing measurements. Also, as used herein, the term "about" generally means within 10%, 5%, 1%, or 0.5% of a given value or range. Alternatively, the term "about" means within an acceptable standard error of the mean when considered by one of ordinary skill in the art. Other than in the operating/working examples, or unless otherwise expressly specified, all of the numerical ranges, amounts, values and percentages such as those for quantities of materials, durations of times, temperatures, operating conditions, ratios of amounts, and the likes thereof disclosed herein should be understood as modified in all instances by the term "about." Accordingly, unless indicated to the contrary, the numerical parameters set forth in the present disclosure and attaching claims are approximations that can vary as desired. At the very least, each numerical parameter should at least be construed in light of the number of reported significant digits and by applying ordinary rounding techniques. Ranges can be expressed herein as from one endpoint to another endpoint or between two endpoints. All ranges disclosed herein are inclusive of the endpoints, unless specified otherwise.

The term "mixing" as used herein refers to the action of adding one solvent/solution to another solvent/solution, without been bound by any particular sequence of which solvent/solution is added first. In one example, a solvent is added to a solution comprising an active pharmaceutical ingredient (API) (e.g., paliperidone palmitate). In another example, the solution comprising the API is added to the solvent.

As used herein, an "excipient" is one that is suitable for use in a subj ect without adverse side effects (such as toxicity, irritation, and allergic response) while commensurate with a reasonable benefit/risk ratio. Also, each excipient must be "acceptable" in the sense of being compatible with the other ingredients of the particles of the present disclosure.

As used herein, the term "active ingredient" or "active pharmaceutical ingredient" refers to, anti-depressants (e.g., paliperidone, and paliperidone palmitate), or anti-oxidant (e.g., L-ascorbic acid).

### 2. Description of preferred embodiments

The present invention discloses an improved method for producing particles of an active ingredient, particularly nanoparticles and/or microparticles of the active ingredient. The thus produced therapeutic particles are not only easier to handle, but also exhibits improved properties, such as improved solubility, stability, and pharmacokinetics.

The present method produces therapeutic particles by taking advantage in the differences between miscibility or solubility of the active ingredient in two miscible solvent systems, so that the active ingredient soluble in a first solvent system precipitates when a second solvent is introduced therein.

Accordingly, to produce particles of the active ingredient, the active ingredient is mixed with the first solvent until it reaches complete dissolution thereby forming a first solution. Then, a second solvent is introduced into the first solution so that particles of the active ingredient are precipitated out of the first solution and thereby forming an emulsified solution. According to preferred embodiments of the present disclosure, the first and second solvents are miscible with each other, while the second solvent is immiscible with the first solution.

Suitable first and second solvents that may be used in the present method are those with solvent polarity index to water ranges between 0.001 and 1.000; such as 0.001, 0.002, 0.003, 0.004, 0.005, 0.006, 0.007, 0.008, 0.009, 0.010 0.011, 0.021, 0.031, 0.041, 0.051, 0.061, 0.071, 0.081, 0.091, 0.101, 0.111, 0.121, 0.131, 0.141, 0.151, 0.161, 0.171, 0.181, 0.191, 0.201, 0.211, 0.221, 0.231, 0.241, 0.251, 0.261, 0.271, 0.281, 0.291, 0.301, 0.311, 0.321, 0.331, 0.341, 0.351, 0.361, 0.371, 0.381, 0.391, 0.401, 0.411, 0.421, 0.431, 0.441, 0.451, 0.461, 0.471, 0.481, 0.491, 0.501, 0.511, 0.521, 0.531, 0.541, 0.551, 0.561, 0.571, 0.581, 0.591, 0.601, 0.611, 0.621, 0.631, 0.641, 0.651, 0.661, 0.671, 0.681, 0.691, 0.701, 0.711, 0.721, 0.731, 0.741, 0.751, 0.761, 0.771, 0.781, 0.791, 0.801, 0.811, 0.821, 0.831, 0.841, 0.851, 0.861, 0.871, 0.881, 0.891, 0.901, 0.911, 0.921, 0.931, 0.941, 0.951, 0.961, 0.971, 0.981, 0.991 and 1.000. Preferably, the solvent polarity index of the first solvent is 0.050-0.4000, such as, 0.050, 0.055, 0.065, 0.075, 0.085, 0.095, 0.105, 0.115, 0.125, 0.135, 0.145, 0.155, 0.165, 0.175, 0.185, 0.195, 0.205, 0.215, 0.225, 0.235, 0.245, 0.255, 0.265, 0.275, 0.285, 0.295, 0.305, 0.315, 0.325, 0.335, 0.345, 0.355, 0.365, 0.375, 0.385, 0.395 and 0.040. More preferably, the solvent polarity index of the first solvent is 0.050-0.350, such as 0.050, 0.060, 0.070, 0.080, 0.090, 0.100, 0.110, 0.120, 0.130, 0.140, 0.150, 0.160, 0.170, 0.180, 0.190, 0.200, 0.210, 0.220, 0.230, 0.240, 0.250, 0.260, 0.270, 0.280, 0.290, 0.300, 0.310, 0.320, 0.330, 0.340 or 0.350.

According to embodiments to produce the paliperidone palmitate particles of the present disclosure, among the solvents that were tested, only 2 solvents (i.e., toluene and tetrahydrofuran) may be used as the first solvent in the present disclosure. Further, among the solvents that were tested, only 3 solvents (i.e., water, heptane, and hexane) are qualified as the second solvent in the present method. According to a non-claimed example, the first solvent is tetrahydrofuran, and the second solvent is water. In a preferred example, the first solvent is toluene, and the second solvent is heptane or hexane.

According to optional embodiments, the emulsified solution described above is filtered and subsequently dried to produce powders of particles of the active ingredient. Each particles of the paliperidone palmitate produced by the method disclosed herein is about 0.1µm to 10µm in diameter. Thus, the particles have a narrow particle size distribution.

To identify suitable solvents for use in the present method, various types of solvents and combinations thereof were tested. Among them, only 3 solvents (i.e., methanol, ethanol, and tetrahydrofuran) are suitable for use as the first solvent in the present method, and only 2 solvents (i.e., water and heptane) are qualified as the second solvent in the present method. According to one preferred example, the first solvent is tetrahydrofuran and the second solvent is heptane. In another preferred example, the first solvent is methanol and the second solvent is water. In one specific example, each therapeutic particles (i.e., the L-ascorbic acid particles) produce by the present method is 7.8 to 47.48 µm in diameter. Compared with the L-ascorbic acid of raw material, the L-ascorbic acid particles provided by present method has a narrower particle size distribution.

Further, due to the relatively narrower size distribution, the particles produced by the present invention exhibit improved properties that reflect on solubility, stability, and/or pharmacokinetics, as compared to those that rare relatively larger in size.

As could be appreciated, the particles of the present invention may be formulated into a pharmaceutical composition with pharmaceutically acceptable carriers, and can be administered to a subject orally or parenterally in various dosage forms. Parenteral administration includes, for example, administration by intraveneous, subcutaneous, intramuscular, transdermal, intrarectal, transnasal, and instillation methods.

The dosage form of the pharmaceutical composition for oral administration includes, for example, tablets, pills, granules, powders, solutions, suspensions, syrups or capsules. As a method of producing a tablet, a pill, granule or powder, it can be formed by conventional techniques using a pharmaceutically acceptable carrier such as excipient, binder, or disintegrant and etc. As to the form of a solution, suspension or syrup, it can be produced by conventional techniques using glycerol esters, alcohols, water or vegetable oils, and etc. The form of capsule can be produced by filling a capsule made of gelatin with the granule, powder or a solution prepared as described above. Among the agents for parenteral administration, in the case of intravenous, subcutaneous or intramuscular administration, it can be administered as injection. An injection can be prepared by dissolving the nanoparticles and/or microparticles of the present invention in water soluble solution such as physiological saline, or water insoluble solution consisting of organic esters such as propylene glycol, polyethylene glycol, or vegetable oils (e.g., sesame oil). In the case of transdermal administration, for example, a dosage form as an ointment or a cream can be employed. The ointment can be produced by mixing the nanoparticle and/or microparticles of the present invention with fats or oils and etc; and the cream can be produced by mixing the nanoparticle and/or microparticles of the present invention with emulsifiers. In the case of rectal administration, it may be in the form of suppository using a gelatin soft capsule. In the case of transdermal administration, it may be in a form of a liquid or a powdery formulation. In a liquid formulation, water, salt solution, phosphate buffer, acetate buffer and the like may be used as a base; it may also contain surfactants, antioxidants, stabilizers, preservatives or tackifiers. In a powdery formulation, it may contain water-absorbing materials such as water-soluble polyacrylates, cellulose low-alkyl esters, polyethylene glycol polyvinyl pyrrolidone, amylase, etc., and non-water absorbing materials such as cellulose, starches, gums, vegetable oils or cross-linked polymers. Further, antioxidants, colorants, preservatives may be added to the powdery formulation. The liquid or powdery formulation may be administered by use of a spray apparatus. In case of inhalation through nose or mouth, a solution or suspension containing the particle of the present disclosure and a pharmaceutical excipient generally accepted for this purpose is inhaled through an inhalant aerosol spray. Alternatively, the particle of the present disclosure in the form of a powder may be administered through inhalator that allows direct contact of the powder with the lung. To these formulations, if necessary, pharmaceutically acceptable carriers such as isotonic agents, preservatives, dispersions, or stabilizers may be added. Further, if necessary, these formulations may be sterilized by filtration, or by treatment with heat or irradiation.

The following examples are provided to elucidate certain aspects of the present invention and to aid those of skilled in the art in practicing this invention. These Examples are in no way to be considered to limit the scope of the invention in any manner. Without further elaboration, it is believed that one skilled in the art can, based on the description herein, utilize the present invention to its fullest extent.

### EXAMPLES

### Example 1 Production and Characterization of paliperidone palmitate particles

### 1.1 The solubility test of paliperidone palmitate in various types of solvents

The solubility of paliperidone palmitate in various types of solvents was evaluated. To this purpose, paliperidone palmitate and the designated solvent were mixed in a ratio of 1/15 (v/w) at 30°C and the mixture was stirred for about 0.5 hour. Results are summarized in Table 1.

**Table 1 Solubility of paliperidone palmitate in various types of solvents**

| First Solvent | Solvent Polarity Index | Solubility of paliperidone palmitate |
|---|---|---|
| Water | 1.000 | insoluble |
| EtOH | 0.654 | insoluble |
| IPA | 0.546 | insoluble |
| ACN | 0.460 | insoluble |
| Acetone | 0.355 | insoluble |
| DCM | 0.309 | soluble |
| EtOAc | 0.028 | insoluble |
| THF | 0.207 | soluble |
| MTBE | 0.124 | insoluble |
| Toluene | 0.099 | soluble |
| Heptane | 0.012 | insoluble |
| Hexane | 0.009 | insoluble |

| | | |
|---|---|---|
| ACN: Acetonitrile; DCM: Dichloromethane ; EtOAc: Ethyl acetate; EtOH: ethanol; IPA: Isopropyl alcohol; MTBE: Methyl tert-butyl ether; THF: tetrahydrofuran | | |

Among the 12 solvents that were tested, paliperidone palmitate was found to be soluble only in DCM, THF, and toluene, and insoluble in the rest of solvents. Thus, DCM, THF and toluene were independently chosen as the first solvent for subsequent production process.

### 1.2 Emulsion test of paliperidone palmitate in various types of solvents

Paliperidone palmitate was dissolved in DCM, THF, or toluene at 30°C and stirred for about 0.5 hour to form a first solution, then a second solvent (as indicated in Table 2) was added and stirred for 3 hr. As the data in Table 2 indicated, among the 36 combinations (i.e., the combination of one of the 3 first solutions and one of the second solvents), only three combinations gave satisfied emulsified solutions, which comprised particles of paliperidone palmitate. The 3 combinations were THF/water, toluene/heptane, and toluene/hexane, respectively.

**Table 2 Solubility of the second solvent in the first solution containing paliperidone palmitate**

| | Paliperidone palmitate dissolved in the first solvent | | |
|---|---|---|---|
| The Second solvent | DCM | THF | Toluene |
| Water | layered | emulsified | layered |
| EtOH | miscible | miscible | miscible |
| IPA | miscible | miscible | miscible |
| ACN | miscible | miscible | miscible |
| Acetone | miscible | miscible | miscible |
| DCM | - | miscible | miscible |
| EtOAc | miscible | miscible | miscible |
| THF | miscible | - | miscible |
| MTBE | miscible | miscible | miscible |
| Toluene | miscible | miscible | - |
| Heptane | miscible | miscible | emulsified |
| Hexane | miscible | miscible | emulsified |

| | | | |
|---|---|---|---|
| ACN: Acetonitrile; DCM: Dichloromethane ; EtOAc: Ethyl acetate; EtOH: ethanol; IPA: Isopropyl alcohol; MTBE: Methyl tert-butyl ether; THF: tetrahydrofuran | | | |

### 1.3 Production of the particles of paliperidone palmitate using toluene and heptane

### 1.3.1 Process A

Seven batches of paliperidone palmitate particles were respectively prepared in accordance to conditions indicated in Tables 3 and 4. Take batch 2 as an example, a 5-L reactor equipped with a mechanical stirrer was charged with paliperidone palmitate and toluene at the first temperature (i.e., 30°C) and stirred at the first speed (i.e., 293 rpm) until paliperidone palmitate was completely dissolved in toluene, thereby forming a clear solution. Then, heptane was added into the clear solution (temperature was approximately 25°C) and stirred at the second speed (i.e., 326 rpm) to form an emulsified solution. The emulsified solution was further cooled to the second temperature (i.e., 0 to 5°C), and stirred for additional 3 hours until paliperidone palmitate particles were formed. Then, the particles were collected and rinsed with cold heptane (i.e., 0 to 5°C) and dried at ambient temperature under vacuum.

**Table 3 Conditions for the production of paliperidone palmitate particles of batches 1 to 4**

| Batch No. | 1 | 2 | 3 | 4 |
|---|---|---|---|---|
| Reactor | 5L | 5L | 5L | 5L |
| Crude API | 80.0 g | 80.0 g | 80.0 g | 80.0 g |
| Toluene | 1040.8 g | 1040.8 g | 1041 g | 1068.8 g |
| Heptane | 888.02 g | 888.02 g | 888.0 g | 911.9 g** |
| 1^{st} speed (rpm) | 307 | 293 | 294 | 300 |
| 1^{st} Temperature | 30 | 30 | 30 | 32 |
| 2^{nd} speed (rpm) | 335 | 326 | 345 | 318 |
| 2^{nd} | 0-5°C | 0-5°C | 0-5°C | 0-5°C |
| Temperature | | | | |
| Concentration (Crude API g/toluene g) | 0.0769 | 0.0769 | 0.0768 | 0.0769 |
| Weight Ratio of toluene to heptane | 1.171 | 1.171 | 1.171 | 1.172 |

| | | | | |
|---|---|---|---|---|
| **The heptane is pre-heated to 30°C. | | | | |

**Table 4 Conditions for the production of paliperidone palmitate particles of batches 5 to 7**

| \| Batch No. | 5 | 6 | 7 |
|---|---|---|---|
| Reactor | 1L | 1L | 1L |
| Crude API | 20.1 g | 20.1 g | 20.2 g |
| Toluene | 261.5 g | 261.5 g | 262.2 g |
| Heptane | 223.2 g* | 223.2 g* | 223.7 g* |
| 1^{st} speed (rpm) | 160 | 165 | 186 |
| 1^{st} Temperature | 37 | 38 | 42 |
| 2^{nd} speed (rpm) | 160 | 165 | 171 |
| 2^{nd} Temperature | 0-5°C | 0-5°C | 0-5°C |
| Concentration (Crude API g/toluene a) | 0.0769 | 0.0769 | 0.077 |
| Weight Ratio of toluene to Heptane | 1.171 | 1.171 | 1.172 |

| | | | |
|---|---|---|---|
| *The heptanes is pre-chilled to 0°C. | | | |

### 1.3.2 Process B

In this example, heptane was charged into the reactor first, then the clear solution of API (e.g., paliperidone palmitate) and toluene was added to the heptane within the reactor. Four batches of paliperidone palmitate particles were respectively prepared in accordance to conditions designated in Tables 5. Take batch 8 as an example, paliperidone palmitate was added into toluene at the first temperature (i.e., 30°C), and stirred at the first speed (i.e., 169 rpm) until paliperidone palmitate was completely dissolved in toluene, thereby forming a clear solution. A 1-L reactor equipped with a mechanical stirrer was charged with heptane, and the clear solution (temperature was approximately 25°C) was added into the reactor and stirred at the second speed (i.e., 169 rpm) to form an emulsified solution. The emulsified solution was further cooled to the second temperature (i.e., 0 to 5°C), and stirred for additional 3 hours until paliperidone palmitate particles were formed. Then, the particles were collected and rinsed with cold heptane (i.e., 0 to 5°C) and dried at ambient temperature under vacuum.

**Table 5 Conditions for the production of paliperidone palmitate particles of batches 8 to 10**

| Batch No. | 8 | 9 | 10 |
|---|---|---|---|
| Reactor | 1L | 1L | 1L |
| Crude API | 20.3 g | 20.3g | 20.3 g |
| Toluene | 264.9 g | 264.9 g | 264.9 g |
| Heptane | 226.0 g* | 226.0 g* | 226.0 g* |
| 1^{st} speed (rpm) | 169 | 170 | 172 |
| 1^{st} Temperature | 40 | 40 | 40 |
| 2^{nd} speed (rpm) | 169 | 170 | 170 |
| 2^{nd} Temperature | 0-5°C | 0-5°C | 0-5°C |
| Concentration (Crude API g/toluene g) | 0.0769 | 0.077 | 0.077 |
| Weight Ratio of toluene to Heptane | 1.171 | 1.172 | 1.172 |

| | | | |
|---|---|---|---|
| *The heptane was pre-chilled to 0°C. | | | |

### 1.4 Production of paliperidone palmitate particles using xylene and heptane (not according to the invention)

The batches 11 to 12 of paliperidone palmitate particles were prepared in accordance to conditions indicated in Table 6. Take batch 11 as an example, a 1-L reactor equipped with a mechanical stirrer was charged with paliperidone palmitate andp-xylene at the first temperature (i.e., 40°C) and stirred at the first speed (i.e., 176 rpm) until paliperidone palmitate was completely dissolved in p-xylene, thereby forming a clear solution. Then, heptane was added into the clear solution (temperature was approximately 25°C) within the reactor and stirred at the second speed (i.e., 176 rpm) to form an emulsified solution. The emulsified solution was further cooled to the second temperature (i.e., 0 to 5°C), and stirred for additional 3 hours until paliperidone palmitate particles were formed. Then, the particles were collected and rinsed with cold heptane (0 to 5°C) and dried at ambient temperature under vacuum.

**Table 6 Conditions for the production of paliperidone palmitate particles of batches 11 and 12**

| Batch No. | 11 | 12 |
|---|---|---|
| Reactor | 1L | 1L |
| Crude API | 23 g | 19.5 g |
| *p*-Xylene | 344.1 g | |
| *o*-Xylene | | 291.6 g |
| Heptane | 254.8 g | 215.9 g |
| 1^{st} speed (rpm) | 176 | 176 |
| 1^{st} Temperature | 40 | 40 |
| 2^{nd} speed (rpm) | 176 | 176 |
| 2^{nd} Temperature | 0-5°C | 0-5°C |
| Concentration (Crude API g/ Xylene g) | 0.0668 | 0.669 |
| Weight Ratio of Xylene to Heptane | 1.35 | 1.35 |

### 1.5 Production of the particles of paliperidone palmitate using toluene and hexane

In this example, another batch 13 of paliperidone palmitate particles was prepared in accordance to conditions indicated in Table 7. A 1-L reactor equipped with a mechanical stirrer was charged with paliperidone palmitate and toluene at the first temperature (i.e., 38°C) and stirred at the first speed (i.e., 160 rpm) until paliperidone palmitate was completely dissolved in toluene, thereby forming a clear solution. Then, n-hexane was added into the clear solution (temperature was approximately 25°C) and stirred at the second speed (i.e., 178 rpm) to form an emulsified solution. The emulsified solution was further cooled to the second temperature (i.e., 0 to 5°C), and stirred for additional 3 hours until paliperidone palmitate particles were formed. Then, the particles were collected and rinsed with cold n-hexane (0 to 5°C) and dried at ambient temperature under vacuum.

**Table 7 Conditions for the production of paliperidone palmitate particles of batch 15**

| Batch No. | 13 |
|---|---|
| Reactor | 1L |
| Crude API | 29.9 g |
| Toluene | 389.1 g |
| n-Hexane | 285.5 g |
| 1^{st} speed (rpm) | 160 |
| 1^{st} Temperature | 68 |
| 2^{nd} speed (rpm) | 178 |
| 2^{nd} Temperature | 0-5°C |
| Concentration (Crude API g/toluene g) | 0.0769 |
| Weight Ratio of toluene to n-Hexane | 1.171 |

| | |
|---|---|
| *The n-hexane is pre-chilled to 0°C. | |

### 1.6 Characterization of the particles of examples 1.1 to 1.5

The paliperidone palmitate particles of Examples 1.1 to 1.5 were subject to particle diameter distribution analysis using a dynamic light scattering particle size distribution analyzer, and the particle size distribution was expressed as D10, D50 and D90. D10, D50 and D90 respectively represent the value of the particle diameter at 10%, 50% and 90% of the particles size distribution. The particle size distribution of paliperidone palmitate particles was as follows: D10: 1.00 to 2.51 µm; D 50: 1.67 to 5.05 µm; D 90: 3.27 to 9.79 µm, and the yield of each size distribution was about 75.5% to 92.44% (see, Table 8 below). The results demonstrated that the particles produced by the present method had a small particle size and a narrow particle size distribution, as compared to that of the control. Further, the present method did not result in the loss of yields, as yields in general remained to be over 75 -92%.

**Table 8 Particle size distribution of paliperidone palmitate particles of batches 1 to 13**

| Batch No. of the sample | D10 (µm) | D50 (µm) | D90 (µm) | Yield |
|---|---|---|---|---|
| 1 | 1.92 | 3.41 | 6.30 | 84.4% |
| 2 | 1.74 | 3.39 | 6.07 | 80.6% |
| 3 | 1.24 | 3.23 | 6.49 | 82.0% |
| 4 | 1.59 | 3.15 | 5.91 | 92.44 |
| 5 | 1.00 | 1.67 | 3.27 | 75.5% |
| 6 | 1.20 | 2.20 | 4.94 | 76.4% |
| 7 | 1.47 | 3.17 | 6.45 | 85.2% |
| 8 | 1.35 | 2.88 | 7.58 | 86.5% |
| 9 | 2.38 | 5.05 | 9.79 | 89.0% |
| 10 | 1.88 | 4.16 | 7.30 | 90.48% |
| 11 | 2.51 | 4.20 | 6.69 | 92.42% |
| 12 | 0.24 | 2.93 | 6.45 | 90.23% |
| 13 | 1.59 | 3.15 | 5.91 | 92.44% |

Furthermore, the crystalline morphology of the paliperidone palmitate particles produced by the present method were further subject to grinding, and the Scanning Electron Microscope (SEM) images taken before and after grinding indicated that grinding did not affect the crystalline structure of the present paliperidone palmitate particles (Figure 1A and Figure 1B), which were all in rectangular-shaped. In addition, it was found that the present paliperidone palmitate particles were easier to grind, as compared to the raw paliperidone palmitate particles (i.e., without being treated by the present method).

### Example 2 Production and Characterization of L-ascorbic acid microparticles

### 2.1 The solubility test of L-ascorbic acid in various types of solvents

The solubility of L-ascorbic acid in various types of solvents was evaluated. To this purpose, L-ascorbic acid and the designated solvent were mixed in a ratio of 1/15 (v/w) at 30°C and stirred for about 0.5 hour. Results are summarized in Table 9.

**Table 9 Solubility of L-ascorbic acid in various types of solvents**

| First Solvent | Solubility of L-ascorbic acid |
|---|---|
| MeOH | Soluble |
| EtOH | Soluble |
| IPA | insoluble |
| ACN | insoluble |
| Acetone | insoluble |
| DCM | insoluble |
| THF | soluble |
| Water | insoluble |
| Toluene | insoluble |
| Heptane | insoluble |

| | |
|---|---|
| MeOH: methanol; EtOH: ethanol; IPA: Isopropyl alcohol; ACN: Acetonitrile; DCM: Dichloromethane; THF: tetrahydrofuran ; EtOAc: Ethyl acetate | |

Among the 10 solvents that were tested, L-ascorbic acid was found to be soluble only in methanol, ethanol, and tetrahydrofuran, and insoluble in the rest of solvents. Thus, methanol, ethanol, and tetrahydrofuran were independently chosen as the first solvent for subsequent production process.

### 2.2 Emulsion test of L-ascorbic acid in various types of solvents

L-ascorbic acid was dissolved in methanol or tetrahydrofuran at 30°C and stirred for about 0.5 hour to form a first solution, then a second solvent (as indicated in Table 9) was added and stirred for 3 hr. Two combinations of solvents gave emulsified solutions, there were tetrahydrofuran/heptane and methanol/water

### 2.3 Production of the L-ascorbic acid particles using methanol and Water

In this example, batch 14 of L-ascorbic acid was prepared. Briefly, a 1-L reactor equipped with a mechanical stirrer was charged with L-ascorbic acid (19.96 g) and methanol (144.8 g) at the first temperature (i.e., 40°C) and stirred at the first speed (i.e., 190 rpm) until L-ascorbic acid was completely dissolved in methanol, thereby forming a clear solution. Then, cold water (i.e., 0°C) was added into the clear solution (temperature was approximately 25°C) and stirred at the second speed (i.e., 185 rpm) to form an emulsified solution. The emulsified solution was further cooled to the second temperature (i.e., 0 to 5°C), and stirred for additional 3 hours until L-ascorbic acid particles were formed. Then, the particles were collected and rinsed with cold water (i.e., 0 to 5°C) and dried at ambient temperature under vacuum.

### 2.4 Production of the L-ascorbic acid particles using tetrahydrofuran and heptane

Batch 15 of L-ascorbic acid was prepared as following. In this example, batch 17 of L-ascorbic acid was prepared. Briefly, a 1-L reactor equipped with a mechanical stirrer was charged with L-ascorbic acid (3.98 g) and tetrahydrofuran (65.1 g) at the first temperature (i.e., 40°C) and stirred at the first speed (i.e., 190 rpm) until L-ascorbic acid was completely dissolved in tetrahydrofuran, thereby forming a clear solution. Then, cold heptane (i.e., 0°C) was added into the clear solution (temperature was approximately 25°C) and stirred at the second speed (i.e., 185 rpm) to form an emulsified solution. The emulsified solution was further cooled to the second temperature (i.e., 0 to 5°C), and stirred for additional 30 min until L-ascorbic acid particles were formed. Then, the particles were collected and rinsed with cold heptane (i.e., 0 to 5°C) and dried at ambient temperature under vacuum.

### 2.5 Characterization of the particles of examples 2.3 to 2.4

The L-ascorbic acid particles of Examples 2.3 to 2.4 were subject to particle diameter distribution analysis using a dynamic light scattering particle size distribution analyzer, and the particle size distribution was expressed as D10, D50 and D90. The particle size distributions of L-ascorbic acid particles of example 2.4 were as follows: D10: 7.8 to 9.3 µm; D 50: 14.86 to 15.99 µm; D 90: 39.34 to 47.48 µm, and the yield of each size distributions was about 97.5% to 99.2% (see, Table 10 below). It is evident that the particles produced by the present method were relatively smaller in size and exhibited a narrower particle size distribution, as compared to those of L-ascorbic acid of raw material (i.e., the control). Further, the present method did not result in the loss of yields, as yields remained to be over 97%, even over 99%.

**Table 10 Particle size distribution of L-ascorbic acid particles of batches 14 and 15**

| Batch No. of the sample | D10 (µm) | D50 (µm) | D90 (µm) | Yield |
|---|---|---|---|---|
| 14 | 9.39 | 15.99 | 39.34 | 99.2% |
| 15 | 7.80 | 14.86 | 47.48 | 97.5% |
| Control | 8.70 | 15.00 | 68.2 | -- |

It will be understood that the above description of embodiments is given by way of example only and that various modifications may be made by those with ordinary skill in the art. The above specification, examples and data provide a complete description of the structure and use of exemplary embodiments of the invention.

## Claims

1. A method for producing particles of an active ingredient comprising:
(a) forming a first solution by dissolving the active ingredient in a first solvent; and
(b) forming the particles of the active ingredient by mixing a second solvent with the first solution to produce an emulsified solution;
wherein, the second solvent is miscible with the first solvent, but is immiscible with the first solution;
wherein either the active ingredient is paliperidone palmitate, the first solvent is toluene and the second solvent is hexane or heptane; or
the active ingredient is ascorbic acid, the first solvent is methanol and the second solvent is water; or the first solvent is tetrahydrofuran and the second solvent is heptane.

2. The method of the claim 1, further comprising:
(c) removing the first and second solvents from the emulsified solution to produce a powder of the particles of the active ingredient.

3. The method of the claim 1, further comprising:
(a-1) filtering the first solution before the step (b).

4. The method of the claim 3, wherein the first solution is filtered through a filter having a pore size of 0.22 µm.

## Patentansprüche

1. Verfahren zur Herstellung von Partikeln eines Wirkstoffs, welches umfasst:
(a) Bilden einer ersten Lösung durch Auflösen des Wirkstoffs in einem ersten Lösungsmittel, und
(b) Bilden der Partikel des Wirkstoffs durch Mischen eines zweiten Lösungsmittels mit der ersten Lösung, um eine emulgierte Lösung zu erzeugen,
wobei das zweite Lösungsmittel mit dem ersten Lösungsmittel mischbar ist, aber mit der ersten Lösung nicht mischbar ist,
wobei entweder der Wirkstoff Paliperidonpalmitat ist, das erste Lösungsmittel Toluol ist und das zweite Lösungsmittel Hexan oder Heptan ist oder
der Wirkstoff Ascorbinsäure ist, das erste Lösungsmittel Methanol ist und das zweite Lösungsmittel Wasser ist; oder das erste Lösungsmittel Tetrahydrofuran ist und das zweite Lösungsmittel Heptan ist.

2. Verfahren nach Anspruch 1, ferner umfassend:
(c) Entfernen des ersten und zweiten Lösungsmittels aus der emulgierten Lösung, um ein Pulver der Partikel des Wirkstoffs herzustellen.

3. Verfahren nach Anspruch 1, ferner umfassend:
(a-1) Filtrieren der ersten Lösung vor Schritt (b).

4. Verfahren nach Anspruch 3, bei welchem die erste Lösung durch einen Filter filtriert wird, welcher eine Porengröße von 0,22 µm aufweist.

## Revendications

1. Procédé de production de particules d'un ingrédient actif comprenant :
(a) la formation d'une première solution par dissolution de l'ingrédient actif dans un premier solvant ; et
(b) la formation de particules de l'ingrédient actif par mélange d'un second solvant avec la première solution pour produire un solution émulsifié ;
dans lequel le second solvant miscible avec le premier solvant, mais est immiscible avec la première solution ;
dans lequel l'ingrédient actif est de la palmite de palipéridone, le premier solvant est du toluène et le second solvant est de l'hexane ou de l'heptane ; ou
l'ingrédient actif est de l'acide ascorbique, le premier solvant est du méthanol et le second solvant est de l'eau ; ou le premier solvant est du tétrahydrofurane et le second solvant est de l'heptane.

2. Procédé selon la revendication 1, comprenant en outre :
(c) le retrait des premier et second solvants de la solution émulsifiée pour produire une poudre des particules de l'ingrédient actif.

3. Procédé selon la revendication 1, comprenant en outre :
(a-1) la filtration de la première solution avant l'étape (b).

4. Procédé selon la revendication 3, dans lequel la première solution est filtré par un filtre présentant une taille de pore de 0,22.
